# EUROPÄISCHE PATENTSCHRIFT

(11) **EP 0 601 002 B1**
(45) Veröffentlichungstag und Bekanntmachung des Hinweises auf die Patenterteilung: **22.01.1997**
(21) Anmeldenummer: 92918175.8
(22) Anmeldetag: 26.08.1992
(51) Int. Cl.: C07D 333/58, C07D 333/66, C07D 335/06, C07D 409/06, C07D 409/04, A61K 31/435, A61K 31/38, A61K 31/495

(54) **BICYCLISCHE SULFONE, VERFAHREN ZU IHRER HERSTELLUNG UND DIESE ENTHALTENDE ARZNEIMITTEL**
BICYCLIC SULPHONES, PROCESS FOR PRODUCING THE SAME AND MEDICAMENTS CONTAINING THE SAME
SULPHONES BICYCLIQUES, LEUR PROCEDE DE PREPARATION ET MEDICAMENTS LES CONTENANT

(30) Priorität: 29.08.1991 DE 4128690
(43) Veröffentlichungstag der Anmeldung: 15.06.1994
(73) Patentinhaber: BOEHRINGER MANNHEIM GMBH, 68298 Mannheim (DE)
(72) Erfinder: FRIEBE, Gunar, D-6800 Mannheim 1 (DE); REINHOLZ, Erhard, D-6800 Mannheim 1 (DE); WILHELMS, Henning, D-6941 Weinheim (DE)
(74) Vertreter: Weber, Manfred, Dr.
(86) Internationale Anmeldenummer: EP9201960
(87) Internationale Veröffentlichungsnummer: WO9305034

(56) Entgegenhaltungen:
- EP-A- 0 322 251
- EP-A- 0 408 760
- EP-A- 0 412 939
- US-A- 2 610 183
- DATABASE WPIL Week 9202, Derwent Publications Ltd., London, GB; AN 92-013611

## Beschreibung

Gegenstand der vorliegenden Erfindung sind bicyclische Sulfone, Verfahren zu deren Herstellung und Arzneimittel, die diese Verbindungen enthalten.

Die Erfindung betrifft bicyclische Sulfone der allgemeinen Formel I 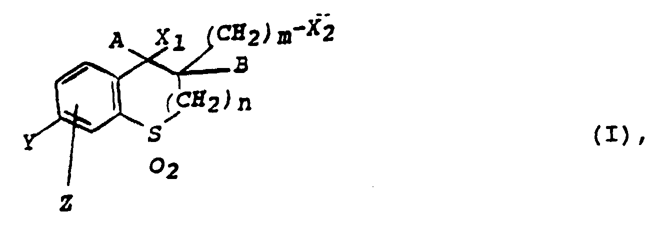 in der
- Y: ein Halogenatom,
- Z: ein Halogenatom oder Wasserstoff,
- m: eine ganze Zahl 1,
- n: eine ganze Zahl 0,
- A und B: jeweils Wasserstoff oder gemeinsam eine Valenzbindung,
- X₁ und X₂: jeweils NR₁R₂ oder, wenn A und B eine Valenzbindung darstellen, einer der beiden Reste X₁ oder X₂ Wasserstoff und der andere NR₁R₂,
- R₁: einen C₁-C₆-Alkyl-, C₃-C₇-Cycloalkyl-, C₂-C₆-Alkenyl- oder einen gewünschtenfalls durch Halogen, C₁-C₆-Alkyl, C₁-C₆-Alkoxy oder Hydroxy substituierten Benzylrest und
- R₂: Wasserstoff oder einen Rest aus der Definition von R₁ bedeuten oder
- R₁ und R₂: gemeinsam mit dem Stickstoffatom einen heterocyclischen Ring bilden, der gewünschtenfalls weitere Heteroatome enthalten und/oder durch C₁-C₆-Alkyl, C₁-C₆-Alkoxy, C₁-C₆-Alkanoyl oder Benzhydryl substituiert sein kann,
deren Tautomere, Enantiomere, Diastereomere und physiologisch verträgliche Salze.

Die neuen Verbindungen der allgemeinen Formel I weisen wertvolle pharmakologische Eigenschaften auf, insbesondere können sie die antigenbedingte Kontraktion von Lungengewebestreifen hemmen. Sie eignen sich daher zur Behandlung allergischer Krankheiten sowie von entzündungsbedingten bronchospastischen und bronchokonstriktorischen Reaktionen.

Die Alkylreste in den genannten Gruppen können geradkettig oder verzweigt sein. Bevorzugte Alkylreste sind der Methyl-, Ethyl-, n-Propyl-, Isopropyl-, n-Butyl-, Isobutyl-, tert.-Butyl-, n-Pentyl- und 3-Pentylrest.

Alkoxy bedeutet vorzugsweise Methoxy oder Ethoxy. Ein Alkenylrest ist vorzugsweise Allyl.

Halogenatome sind insbesondere Fluor, Chlor und Brom.

Cycloalkylreste bedeuten vorzugsweise Cyclopropyl, Cyclobutyl, Cyclopentyl, Cyclohexyl oder Cycloheptyl, insbesondere Cyclopentyl.

Heterocyclische Ringe sind beispielsweise Pyrrolidin, Piperidin, Morpholin, Thiomorpholin, Piperazin und Homopiperidin.

Außer den in den Beispielen genannten Verbindungen sind Gegenstand der Erfindung insbesondere alle Substanzen, die jede mögliche Kombination der in den Beispielen genannten Substituenten aufweisen.

Das erfindungsgemäße Verfahren zur Herstellung der Verbindungen der Formel I ist dadurch gekennzeichnet, daß man in an sich bekannter Weise eine Verbindung der allgemeinen Formel II 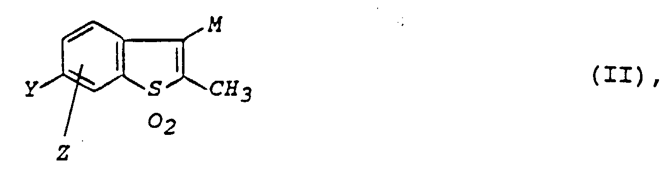 in welcher Y und Z die angegebenen Bedeutung haben und M einen reaktiven Rest darstellt,
mit einer Verbindung der allgemeinen Formel III

HNR₁R₂ (III),

in welcher R₁ und R₂ die angegebene Bedeutung haben,
umsetzt und anschließend gewünschtenfalls die erhaltenen Verbindungen der Formel I durch Umsetzung mit physiologisch verträglichen Säuren in ihre Salze umwandelt.

Als reaktive Reste M kommen Chlor und Brom in Frage.

Die Umsetzung von Verbindungen der Formel II mit Verbindungen der Formel III erfolgt zweckmäßig in einem Lösungsmittel wie beispielsweise einem niederen Alkohol wie Methanol, Ethanol oder Isopropanol oder einem Ether wie Tetrahydrofuran oder einem Amin wie Pyridin. Es kann jedoch auch überschüssige Komponente der Formel III als Solvens Verwendung finden.

Die Ausgangsverbindugnen II und III sind literaturbekannte Substanzen oder können in Analogie zu literaturbekannten Verfahren hergestellt werden. Die Synthese von 3,5-Dichlor-2-methyl-benzo[b]thiophen-1,1-dioxid ist in J. Heterocycl. Chem. 3, 174 (1966) beschrieben.

Unter den jeweiligen Reaktionsbedingungen entstehen Produktgemische, deren Aufarbeitung entweder zwei unterschiedliche Produkte oder nur ein Hauptprodukt liefert. Die erhaltenen Produkte sind durch physikalische Daten (Schmelzpunkte, IR und NMR-Daten) eindeutig charakterisiert.

Als pharmakologisch verträgliche Salze kommen insbesondere Salze mit nichttoxischen anorganischen oder organischen Säuren wie z.B. Salzsäure, Schwefelsäure, Phosphorsäure, Bromwassersäure, Essigsäure, Milchsäure, Zitronensäure, Äpfelsäure, Benzoesäure, Salicylsäure, Malonsäure, Maleinsäure, Bernsteinsäure oder Diaminocapronsäure in Frage.

Die Salze erhält man in üblicher Weise z.B. durch Neutralisation der Verbindungen der Formel I mit den entsprechenden Säuren.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und / oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Gemschmacks- und Süßstoffe enthalten. Für die äußerliche Anwendung können die erfindungsgemäßen Substanzen I auch in Form von Pudern und Salben verwendet werden. Sie werden dazu z.B. mit pulverförmigen, physiologisch verträglichen Verdünnungsmitteln bzw. üblichen Salbengrundlagen vermischt.

Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht.

Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Außer den in den Beispielen genannten Substanzen sind im Sinne der vorliegenden Erfindung die folgenden Verbindungen bevorzugt:
1. 6-Chlor-2-(4-thiomorpholino-methyl)benzo[b]thiophen-1,1-dioxid
2. 6-Chlor-2-(N-butylamino-methyl)benzo[b]thiophen-1,1-dioxid
3. 6-Chlor-2-(N-cyclopentylamino-methyl)benzo[b]thiophen-1,1-dioxid
4. 6-Chlor-2-(N-methylamino-methyl)benzo[b]thiophen-1,1-dioxid
5. 6-Chlor-2-methyl-3-diethylamino-benzo[b]thiophen-1,1-dioxid
6. 6-Chlor-2-methyl-3-pyrrolidino-benzo[b]thiophen-1,1-dioxid
7. 6-Chlor-2-methyl-3-dimethylamino-benzo[b]thiophen-1,1-dioxid
8. 6-Chlor-2-nethyl-3-piperidino-benzo[b]thiophen-1,1-dioxid
9. 6-Chlor-2-methyl-3-(4-methoxy-piperidino)benzo[b]thiophen-1,1-dioxid
10. 6-Chlor-2-methyl-3-hexanethyleninino-benzo[b]thiophen-1,1-dioxid
11. 6-Chlor-2-methyl-3-(4-thiomorpholino)benzo[b]thiophen-1,1-dioxid
12. 6-Chlor-2-methyl-3-N,N-diallylamino-benzo[b]thiophen-1,1-dioxid

### Beispiel 1

### 6-Chlor-2-(N,N-diethylamino-methyl)benzo[b]thiophen-1,1-dioxid

Eine Mischung aus 37.3 g (0.15 mol) 3,6-Dichlor-2-methylbenzo[b]thiophen-1,1-dioxid, 200 ml Methanol und 45 ml Diethylamin wird 4 h zum Rückfluß erhitzt. Darauf engt man ein, nimmt in Ethylacetat auf, wäscht mit Wasser, trocknet, engt ein und chromatographiert an Kieselgel (Elutionsmittel Ethylacetat/Isohexan 1:1). Man isoliert 29.5 g Titelverbindung (62 % d.Th.) vom Schmp. 58-59°C.

Durch Versetzen der Ethylacetatlösung mit überschüssiger etherischer Chlorwasserstofflösung erhält man das Hydrochlorid vom Schmp. 217-218°C.

### Beispiel 2

### 6-Chlor-2-(4-morpholino-methyl)benzo[b]thiophen-1,1-dioxid und 6-Chlor-2-methyl-3-(4-morpholino)benzo[b]thiophen-1,1-dioxid

Eine Mischung aus 5.0 g (20 mmol) 3,6-Dichlor-2-methylbenzo[b]thiophen-1,1-dioxid, 40 ml Methanol und 6.5 ml Morpholin wird 24 h zum Rückfluß erhitzt. Darauf engt man ein und chromatographiert an Kieselgel (Elutionsmittel Ethylacetat/Isohexan 1:1). Man isoliert als erste Fraktion 1.4 g 6-Chlor-2-methyl-3-(4-morpholino)benzo[b]thiophen-1,1-dioxid (23 % d.Th.) vom Schmp. 220-222°C und als zweite Fraktion 1.6 g 6-Chlor-2-(4-morpholino-methyl)benzo[b]thiophen-1,1-dioxid (27 % d.Th.) vom Schmp. 127-129°C.

### Beispiel 3

In analoger Weise wie in Beispiel 1 oder 2 beschrieben isoliert man aus dem Produktgemisch der Umsetzung von 3,6-Dichlor-2-methyl-benzo[b]thiophen-1,1-dioxid und dem jeweiligen Amin:

| Bezeichnung | | Ausb. % | Schmelzpunkt °C (Lsg.mittel) |
|---|---|---|---|
| a) | 7-Chlor-3,4-bis-pyrrolidinobenzo[b]thian-1,1-dioxid aus Pyrrolidin (Referenz-Verbindung) | 15 | 70-71 (Isohexan) |
| b) | 6-Chlor-2-pyrrolidinomethylbenzo[b]thiophen-1,1-dioxid aus Pyrrolidin | 37 | 192-194 (Ethanol) |
| c) | 6-Chlor-2-dimethylaminomethyl-benzo[b]thiophen-1,1-dioxid aus Dimethylamin | 21 | 173-174 (Ethylacetat) |

### Beispiel 4

### 6-Chlor-2-piperidinomethyl-benzo[b]thiophen-1,1-dioxid

Eine Mischung aus 3.75 g 3,6-Dichlor-2-methylbenzo[b]thiophen-1,1-dioxid und 35 ml Piperidin wird 4 h bei 80°C gerührt, anschließend mit Wasser versetzt und mit Ethylacetat extrahiert. Den Extrakt engt man ein und verreibt ihn mit Ethylacetat. Man isoliert 1.6 g Titelverbindung (36 % d.Th.) vom Schmp. 154-156°C.

### Beispiel 5

In analoger Weise wie in Beispiel 4 beschrieben isoliert man aus dem Produktgemisch der Umsetzung von 3,6-Dichlor-2-methyl-benzo[b]thiophen-1,1-dioxid und dem jeweiligen Amin, gegebenenfalls nach chromatographischen Trennung:

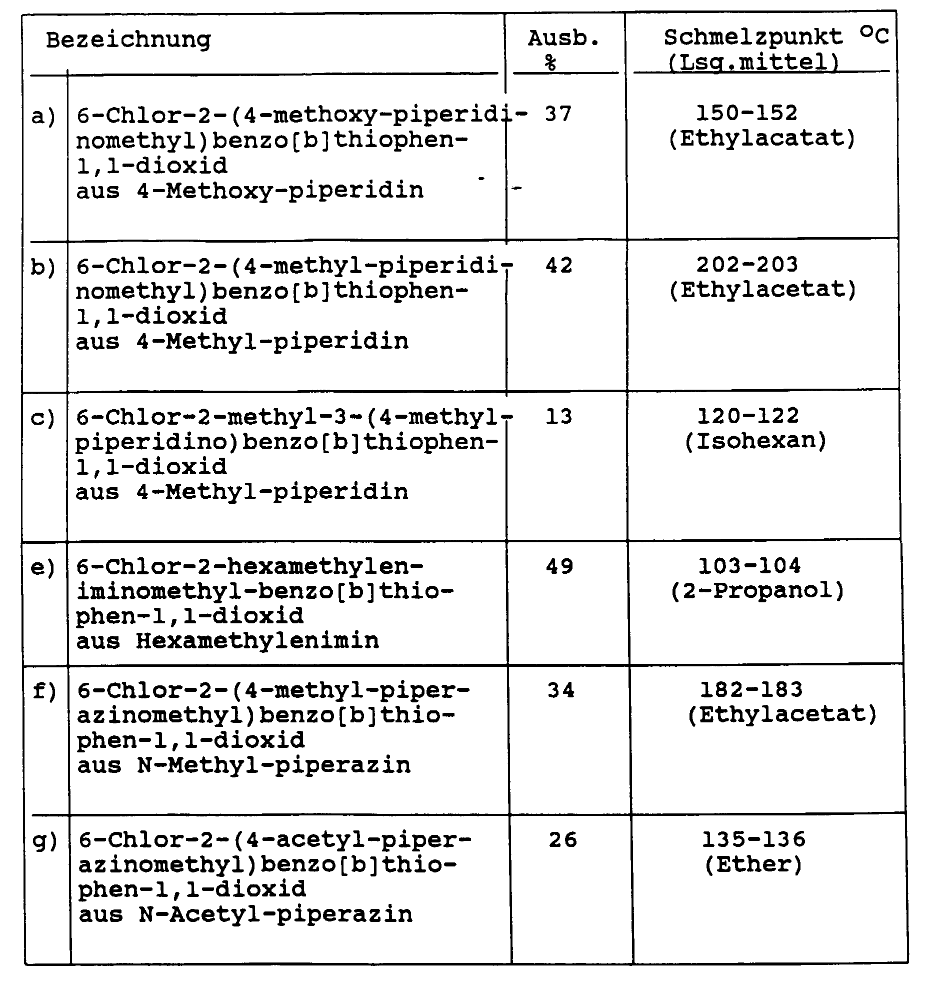

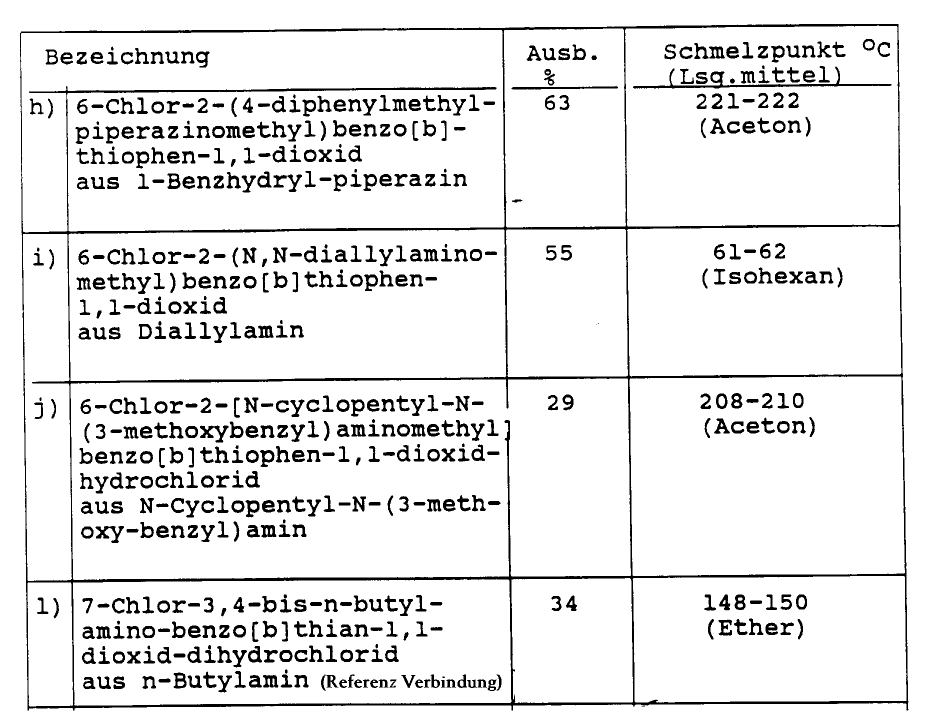

### Beispiel 6

### 5,6-Dichlor-2-(N,N-diethylamino-methyl)benzo[b]thiophen-1,1-dioxid-hydrochlorid

In analoger Weise wie in Beispiel 1 beschrieben erhält man aus 3,5,6-Trichlor-2-methyl-benzo[b]thiophen-1,1-dioxid und Diethylamin in 22 % Ausbeute die Titelverbindung als Hydrochlorid vom Schmp. 247-248°C (aus Ethylacetat).

### Beispiel 7

### 6,7-Dichlor-2-(N,N-diethylamino-methyl)benzo[b]thiophen-1,1-dioxid-hydrochlorid

In analoger Weise wie in Beispiel 1 beschrieben erhält man aus 3,6,7-Trich1or-2-methyl-benzo[b]thiophen-1,1-dioxid und Diethylamin in 19 % Ausbeute die Titelverbindung als Hydrochlorid vom Schmp. 216-218°C (aus Ethylacetat).

Die als Ausgangsstoffe für Beispiel 6 und 7 eingesetzten Trichlor-methyl-benzo[b]thiophen-1,1-dioxide können wie folgt erhalten werden:

Zu 24 ml Chlorsulfonsäure gibt man portionsweise 10.0 g (0.05 mol) 3,4-Dichlor-propiophenon, rührt 3 h bei 100°C, gießt auf Eis, filtriert, rührt den Niederschlag mit 50 ml konz. Ammoniak aus, filtriert, wäscht mit Wasser und chromatographiert den Rückstand an Kieselgel (Elutionsmittel Isohexan/Ethylacetat 9:1). Man isoliert 6.8 g 3,5,6-Trichlor-2-methylbenzo[b]thiophen-1,1-dioxid (48 % d.Th.) vom Schmp. 194-196°C sowie 2.9 g 3,6,7-Trichlor-2-methyl-benzo[b]thiophen-1,1-dioxid (20 % d.Th.) vom Schmp. 179-181°C.

### Prüfbericht

### Hemmung der Antigen-bedingten Konstriktion von passiv sensibilisierten Meerschweinchen - Lungenparenchym - Streifen in vitro (Organbad)

Für die in vitro Untersuchung der erfindungsgemäßen Verbindungen wurde die Hemmung der Antigen-bedingten Konstriktion von passiv sensibilisiertem Meerschweinchen-Lungenparenchym-Streifen gemessen, wie nachfolgend beschrieben:

Pirbright-White Meerschweinchen wurden betäubt durch Genickschlag und entblutet. Die Lungen wurden in situ mit Krebs-Puffer, pH 7.4, weitgehend blutfrei gespült. Anschließend wurde die Lunge entnommen, in Streifen geschnitten (ca. 20 x 4 x 4 mm), und die Streifen für eine Stunde bei Raumtemperatur mit einer 1:50-Verdünnung eines homologen anti-Ov-Albumin-Antiserums passiv sensibilisiert und dann mit Krebs-Puffer 1 x gewaschen. Das Antiserum war vorher nach DAVIES (1) in Meerschweinchen des gleichen Stammes erzeugt worden durch wiederholte Injektion von Ov-Albumin (2 x kristallisiert) unter Zusatz von komplettem Freund'schen Adjuvans. Bis zu seiner Verwendung wurde das Antiserum bei - 18 °C unverdünnt gelagert. Anschließend wurden die Lungenstreifen einzeln in 10 ml-Wasserbädern mit einer Vorspannung von 1.2 g an einem isometrischen Meßaufnehmer aufgehängt. Danach wurden die Bäder mit Krebs-Puffer gefüllt und kontinuierlich bei 37 °C mit O₂ (95 %) und CO₂ (5 %) begast. Über einen Verstärker wurden die Konstriktionen der Lungenstreifen auf einem Schreiber aufgezeichnet. Nach 30-minütiger Eingewöhnungsphase wurden Histamin-Kontrollspasmen zur Erkennung der Reaktionsfähigkeit der Organstücke erzeugt, gewaschen, anschließend für 20 Minuten die Prüfsubstanz bei 37 °C vorinkubiert und danach die Ov-Albumin-bedingte Konstriktion ausgelöst. Die Hemmwirkungen der erfindungsgemäßen Verbindungen wurden als prozentuale Verringerung der Konstriktionsamplitude der "Proben mit Prüfsubstanz" im Verhältnis zu den "ungehandelten Kontrollkonstriktionen" ausgedrückt.

### (1) DAVIES, G.E. T.P. Johnstone

Quantitative studies on anaphylaxis in guinea pigs passively sensitized with homologous antibody. Inter. Arch. Allergy 41, 648 - 454 (1971)

**Tabelle**

| % Hemmung der durch Ovalbumin (0,1 µg/ml) induzierten Konstriktion von passiv-sensibilisiertem Lungenparenchynstreifen (Meerschweinchen) | | |
|---|---|---|
| 20 min/3.7 °C Vorinkubationszeit (Organbadtechnik) | | |
| n = Anzahl der Tests | | |
| * = 200 µg/ml | | |
| Substanz Bsp.No. | Konzentration (20 ug/ml) | n |
| Aminophyllin | 26* | 6 |
| 1 | 53 | 2 |
| 3a) | 25 | 3 |
| 6 | 51 | 3 |
| 7 | 88 | 3 |
| 51) | 25 | 3 |

## Patentansprüche

1. Bicyclische Sulfone der Formel I 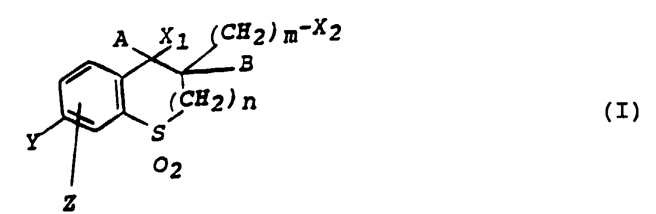 in der
Y ein Halogenatom,
Z ein Halogenatom oder Wasserstoff,
m eine ganze zahl 1,
n eine ganze Zahl 0,
A und B jeweils Wasserstoff oder gemeinsam eine Valenzbildung
X₁ und X₂ jeweils NR₁R₂ oder, wenn A und B eine Valenzbindung darstellen, einer der beiden Reste X₁ oder X₂ Wasserstoff und der andere NR₁R₂,
R₁ einen C₁-C₆-Alkyl-, C₃-C₇-Cycloalkyl-, C₂-C₆-Alkenyl-oder einen gewünschtenfalls durch Halogen, C₁-C₆-Alkyl, C₁-C₆-Alkoxy oder Hydroxy substituierten Benzylrest und
R₂ Wasserstoff oder einen Rest aus der Definition von R₁ bedeuten oder
R₁ und R₂ gemeinsam mit dem Stickstoffatom einen heterocyclischen Ring bilden, der gewünschtenfalls weitere Heteroatome enthalten und/oder durch C₁-C₆-Alkyl, C₁-C₆-Alkoxy, C₁-C₆-Alkanoyl oder Benzhydryl substituiert sein kann,
deren Tautomere, Enantiomere, Diastereomere und physiologisch verträgliche Salze.

2. Verfahren zur Herstellung von bicyclischen Sulfonen der Formel I 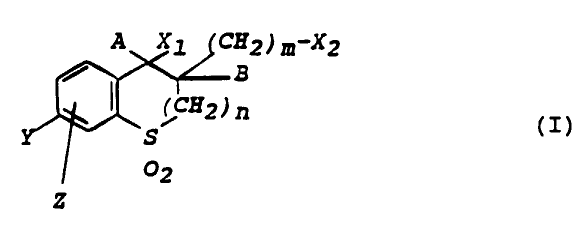 in der
Y ein Halogenatom,
Z ein Halogenatom oder Wasserstoff,
m eine ganze Zahl 1,
n eine ganze Zahl 0,
A und B jeweils Wasserstoff oder gemeinsam eine Valenzbildung
X₁ und X₂ jeweils NR₁R₂ oder, wenn A und B eine Valenzbindung darstellen, einer der beiden Reste X₁ oder X₂ Wasserstoff und der andere NR₁R₂,
R₁ einen C₁-C₆-Alkyl-, C₃-C₇-Cycloalkyl-, C₂-C₆-Alkenyloder einen gewunschtenfalls durch Halogen, C₁-C₆-Alkyl, C₁-C₆-Alkoxy oder Hydroxy substituierten Benzylrest und
R₂ Wasserstoff oder einen Rest aus der Definition von R₁ bedeuten oder
R₁ und R₂ gemeinsam mit dem Stickstoffatom einen heterocyclischen Ring bilden, der gewünschtenfalls weitere Heteroatome enthalten und/oder durch C₁-C₆-Alkyl, C₁-C₆-Alkoxy, C₁-C₆-Alkanoyl oder Benzhydryl substituiert sein kann,
deren Tautomere, Enantiomere, Diastereomere und physiologisch verträgliche Salze,
dadurch gekennzeichnet, daß man in an sich bekannter Weise eine Verbindung der allgemeinen Formel II 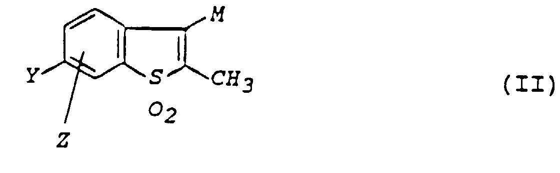 in welcher Y und Z die angegebenen Bedeutung haben und M einen reaktiven Rest darstellt,
mit einer Verbindung der allgemeinen Formel III
HNR₁R₂ (III),
in welcher R₁ und R₂ die angegebene Bedeutung haben,
umsetzt und anschließend gewünschtenfalls die erhaltenen Verbindungen der Formel I durch Umsetzung mit physiologisch verträglichen Säuren in ihre Salze umwandelt.

3. Arzneimittel, enthaltend mindestens eine Verbindung gemäß Anspruch 1 neben üblichen Träger- und Hilfsstoffen.

4. Verwendung von Verbindungen gemäß Anspruch 1 zur Herstellung von Arzneimitteln zur Behandlung von allergischen Erkrankungen.

5. Verwendung von Verbindungen gemäß Anspruch 1 zur Herstellung von Arzneimitteln zur Behandlung von entzündungsbedingten bronchospastischen und bronchokonstriktorischen Reaktionen.

## Claims

1. Bicyclic sulfones of formula I 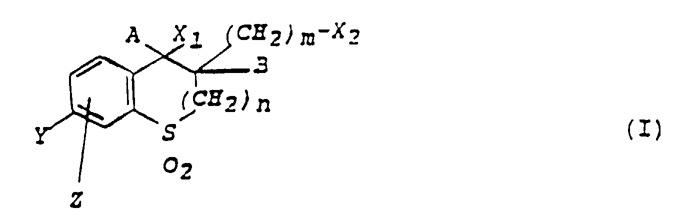 in which
Y is a halogen atom,
Z is a halogen atom or hydrogen,
m is an integer 1,
n is an integer 0,
A and B each represent hydrogen or together represent a valency bond,
X₁ and X₂ each represent NR₁R₂ or, if A and B represent a valency bond, one of the two residues X₁ or X₂ represents hydrogen and the other represents NR₁R₂,
R₁ denotes a C₁-C₆ alkyl, C₃-C₇ cycloalkyl, C₂-C₆ alkenyl or a benzyl residue substituted if desired, by halogen, C₁-C₆ alkyl, C₁-C₆ alkoxy or hydroxy and
R₂ denotes hydrogen or a residue from the definition of R₁ or
R1 and R2 together with the nitrogen atom form a heterocyclic ring which if desired, contains further heteroatoms and/or can be substituted by C₁-C₆ alkyl, C₁-C₆ alkoxy, C₁-C₆ alkanoyl or benzhydryl,
their tautomers, enantiomers, diastereomers and physiologically tolerated salts.

2. Process for the production of bicyclic sulfones of the formula I 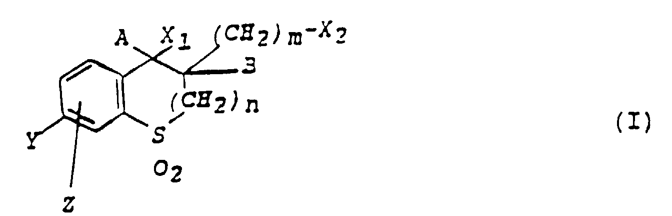 in which
Y is a halogen atom,
Z is a halogen atom or hydrogen,
m is an integer 1,
n is an integer 0,
A and B each represent hydrogen or together represent a valency bond,
X₁ and X₂ each represent NR₁R₂ or, if A and B represent a valency bond, one of the two residues X₁ or X₂ represents hydrogen and the other represents NR₁R₂,
R₁ denotes a C₁-C₆ alkyl, C₃-C₇ cycloalkyl, C₂-C₆ alkenyl or a benzyl residue substituted if desired, by halogen, C₁-C₆ alkyl, C₁-C₆ alkoxy or hydroxy and
R₂ denotes hydrogen or a residue from the definition of R₁ or
R1 and R2 together with the nitrogen atom form a heterocyclic ring which if desired, contains further heteroatoms and/or can be substituted by C₁-C₆ alkyl, C₁-C₆ alkoxy, C₁-C₆ alkanoyl or benzhydryl,
their tautomers, enantiomers, diastereomers and physiologically tolerated salts,
wherein a compound of the general formula II 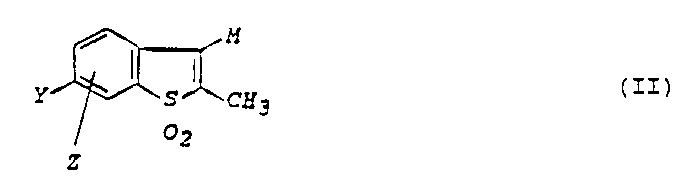 in which Y and Z have the stated meaning and M represents a reactive residue,
is reacted in a known manner with a compound of the general formula III
HNR₁R₂ (III),
in which R₁ and R₂ have the stated meaning,
and subsequently, if desired, the compounds of formula I obtained are converted into their salts by reaction with physiologically tolerated acids.

3. Pharmaceutical agent containing at least one compound as claimed in claim 1 in addition to the usual vehicles and auxiliary substances.

4. Use of compounds as claimed in claim 1 to produce pharmaceutical agents for the treatment of allergic diseases.

5. Use of compounds as claimed in claim 1 to produce pharmaceutical agents for the treatment of bronchospastic and bronchoconstrictory reactions caused by inflammation.

## Revendications

1. Sulfones bicycliques de formule I 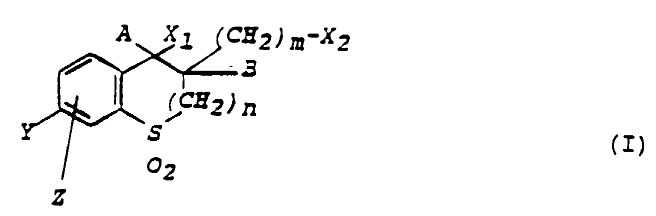 dans laquelle
Y représente un atome d'halogène,
Z représente un atome d'halogène ou d'hydrogène,
m est un nombre entier qui vaut 1,
n est un nombre entier qui vaut 0,
A et B représentent chacun un atome d'hydrogène ou forment en commun une valence libre,
X₁ et X₂ représentent chacun NR₁R₂ ou lorsque A et B représentent une valence libre, l'un des deux restes X₁ et X₂ représente un atome d'hydrogène, et l'autre, NR₁R₂,
R₁ représente un reste alkyle en C₁-C₆, cycloalkyle en C₃-C₇, alcényle en C₂-C₆ ou un reste benzyle éventuellement substitué par un atome d'halogène, un reste alkyle en C₁-C₆, alcoxy en C₁-C₆ ou hydroxy, et
R₂ représente un atome d'hydrogène ou un reste défini comme pour R₁, ou
R₁ et R₂ forment ensemble avec l'atome d'azote un hétérocycle qui peut contenir éventuellement d'autres hétéroatomes et/ou peut être substitué par un reste alkyle en C₁-C₆, alcoxy en C₁-C₆, alcanoyle en C₁-C₆ ou benzhydryle,
leurs tautomères, énantiomères, diastéréomères et sels physiologiquement acceptables.

2. Procédé de préparation de sulfones bicycliques de formule I 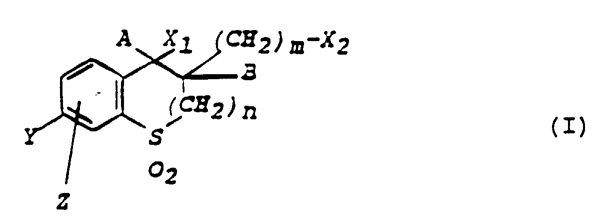 dans laquelle
Y représente un atome d'halogène,
Z représente un atome d'halogène ou d'hydrogène,
m est un nombre entier qui vaut 1,
n est un nombre entier qui vaut 0,
A et B représentent chacun un atome d'hydrogène ou forment en commun une valence libre,
X₁ et X₂ représentent chacun NR₁R₂ ou lorsque A et B représentent une valence libre, l'un des deux restes X₁ et X₂ représente un atome d'hydrogène, et l'autre, NR₁R₂,
R₁ représente un reste alkyle en C₁-C₆, cycloalkyle en C₃-C₇, alcényle en C₂-C₆ ou un reste benzyle éventuellement substitué par un atome d'halogène, un reste alkyle en C₁-C₆, alcoxy en C₁-C₆ ou hydroxy, et
R₂ représente un atome d'hydrogène ou un reste défini comme pour R₁, ou
R₁ et R₂ forment ensemble avec l'atome d'azote un hétérocycle qui peut contenir éventuellement d'autres hétéroatomes et/ou peut être substitué par un reste alkyle en C₁-C₆, alcoxy en C₁-C₆, alcanoyle en C₁-C₆ ou benzhydryle,
leurs tautomères, énantiomères, diastéréomères et sels physiologiquement acceptables, caractérisé en ce que de manière connue en soi, on fait réagir un composé de formule générale II 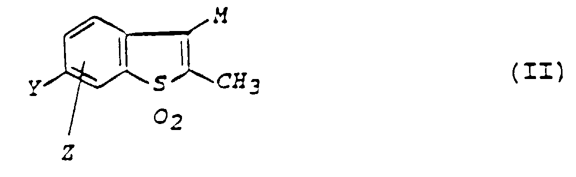 dans laquelle Y et Z ont les significations indiquées et M représente un reste réactif,
avec un composé de formule générale III
HR₁R₂ (III),
dans laquelle R₁ et R₂ ont les significations indiquées,
et ensuite, on transforme éventuellement les composés de formule I obtenus en leurs sels par réaction avec des acides physiologiquement acceptables.

3. Médicament contenant au moins un composé selon la revendication 1 en plus de véhicules et adjuvants usuels.

4. Utilisation de composés selon la revendication 1 pour la préparation de médicaments destinés au traitement de maladies allergiques.

5. Utilisation de composés selon la revendication 1 pour la préparation de médicaments destinés au traitement de réactions bronchospastiques et bronchoconstrictives provoquées par inflammation.
